(19) Europäisches Patentamt
European Patent Office
Office européen des brevets

(11) Veröffentlichungsnummer: **0 340 153**
**A1**

(12) # EUROPÄISCHE PATENTANMELDUNG

(21) Anmeldenummer: **89730085.1**

(22) Anmeldetag: **31.03.89**

(51) Int. Cl.[4]: **C 07 D 233/60**
A 61 K 31/415, C 07 D 233/58

(30) Priorität: **31.03.88 DE 3811574**

(43) Veröffentlichungstag der Anmeldung:
**02.11.89 Patentblatt 89/44**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Strehlke, Peter, Dr.**
**Droysenstrasse 10A**
**D-1000 Berlin 12 (DE)**

**Bohlmann, Rolf, Dr.**
**Kühler Weg 6a**
**D-1000 Berlin 19 (DE)**

**Henderson, David, Dr.**
**Jahnstrasse 17**
**D-1000 Berlin 28 (DE)**

**Nishino, Yukishige, Dr.**
**Lanzendorfer Weg 14**
**D-1000 Berlin 22 (DE)**

(54) **N-substituierte Imidazole, Verfahren zu ihrer Herstellung sowie ihre Verwendung in Arzneimitteln.**

(57) Die Erfindung betrifft N-substituierte Imidazole, der allgemeinen Formel I

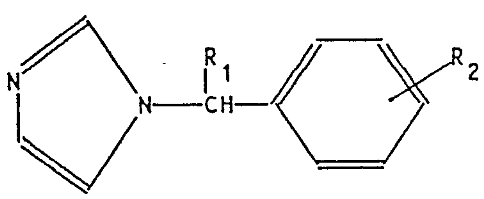

worin
$R_1$ ein Wasserstoffatom, einen gesättigten oder ungesättigten, gerad- oder verzweigtkettigen Kohlenwasserstoffrest, einen cyclischen Kohlenwasserstoffrest, einen Cycloalkylalkylrest oder einen Arylalkylrest,
und
$R_2$ einen substituierten gerad- oder verzweigtkettigen, gesättigten oder ungesättigten Kohlenwasserstoffrest, eine gegebenenfalls derivatisierte Formylgruppe, eine gegebenenfalls substituierte Alkanoylgruppe, eine gegebenenfalls substituierte Benzoylgruppe oder eine gegebenenfalls derivatisierte Carboxylgruppe
bedeutet,

sowie die Verbindungen in Form ihrer Salze, Verfahren zu ihrer Herstellung sowie ihre Verwendung in Arzneimitteln.

Die erfindungsgemäßen Verbindungen besitzen aromatasehemmende Eigenschaften.

EP 0 340 153 A1

**Beschreibung**

## N-SUBSTITUIERTE IMIDAZOLE, VERFAHREN ZU IHRER HERSTELLUNG SOWIE IHRE VERWENDUNG IN ARZNEIMITTELN

Die Erfindung betrifft N-substituierte Imidazole, Verfahren zu ihrer Herstellung sowie ihre Verwendung in Arzneimitteln.

Die neuen N-substituierten Imidazole werden durch die allgemeine Formel I gekennzeichnet

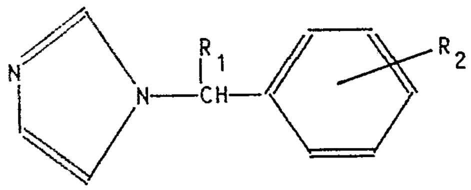

(I),

worin

$R_1$ ein Wasserstoffatom, einen gesättigten oder ungesättigten, gerad- oder verzweigtkettigen Kohlenwasserstoffrest mit 1 bis 10 Kohlenstoffatomen, einen cyclischen Kohlenwasserstoffrest mit 3 bis 9 Kohlenstoffatomen, einen Cycloalkylalkylrest mit 4 bis 12 Kohlenstoffatomen oder einen Arylalkylrest mit 7 bis 10 Kohlenstoffatomen,

und

$R_2$ einen substituierten gerad- oder verzweigtkettigen, gesättigten oder ungesättigten Kohlenwasserstoffrest mit 1 bis 10 Kohlenstoffatomen, eine gegebenenfalls derivatisierte Formylgruppe, eine gegebenenfalls substituierte Alkanoylgruppe mit 2 bis 10 Kohlenstoffatomen, eine gegebenenfalls substituierte Benzoylgruppe oder eine gegebenenfalls derivatisierte Carboxylgruppe

bedeutet.

Die Verbindungen können auch in Form ihrer Salze vorliegen.

Die als Rest $R_1$ infrage kommenden gesättigten oder ungesättigten, gerad- oder verzweigtkettigen Kohlenwasserstoffreste mit 1 bis 10 Kohlenstoffatomen sind beispielsweise der Methyl-, Ethyl-, Propyl-, Propenyl-, Isopropyl-, Butyl-, Hexyl-, Octyl- und Decylrest, wobei der Propylrest bevorzugt ist. Als cyclische Kohlenwasserstoffreste mit 3 bis 9 Kohlenstoffatomen sind beispielsweise der Cyclopentyl- und Cyclohexylrest zu nennen. Als Cycloalkylalkylreste mit 4 bis 12 Kohlenstoffatomen sind beispielsweise der Cyclopentylmethyl- und Cyclohexylmethylrest zu nennen. Als Arylalkylrest mit 7 bis 10 Kohlenstoffatomen ist beispielsweise der Benzylrest zu nennen.

Die als Reste $R_2$ infrage kommenden substituierten gerad- oder verzweigtkettigen gesättigten oder ungesättigten Kohlenwasserstoffreste mit 1 bis 10 Kohlenstoffatomen, sind beispielsweise substituierte Methyl-, Ethyl-, Propyl-, Benzyl-, Phenyl- und Ethenylgruppen. Als Substituenten dieser Gruppen sind beispielsweise Halogen-, Ether- und Aminreste, vorzugsweise Hydroxyl-, Cyano-, Methoxy-, Butoxy-, Phenoxy-, Amino-, Pyrrolidinyl-, Carboxyl-, Carbonsäureester- und Carbonsäureamidreste zu nennen.

Weiter hat $R_2$ die Bedeutung einer gegebenenfalls derivatisierten Formylgruppe, wobei beispielsweise solche Derivate zu nennen sind, die durch Umsetzung der Formylgruppe mit einem Amin oder Aminderivat erhältlich sind. Als vorteilhaft haben sich die Produkte, die durch Umsetzung mit Hydroxylamin, O-Methylhydroxylamin, O-Ethylhydroxylamin, O-Allylhydroxylamin, O-Benzylhydroxylamin, O-4-Nitrobenzyloxyhydroxylamin, O-2,3,4,5,6-Pentafluorbenzyloxyhydroxylamin, Semicarbazid, Thiosemicarbazid, Ethylamin und Anilin erhalten werden können, erwiesen.

Weiter hat $R_2$ die Bedeutung einer gegebenenfalls derivatisierten Carboxylgruppe, wobei als Derivate beispielsweise Carbonsäureester und Carbonsäureamide zu nennen sind. Als besonders geeignet haben sich Methyl-, Ethyl-und Butylester sowie Carbonsäureamid, -isopropylamid, -anilid und -pyrrolidinylamid erwiesen.

Weiter hat $R_2$ die Bedeutung einer gegebenenfalls substituierten Alkanoylgruppe mit 2 bis 10 Kohlenstoffatomen. Beispielsweise kann die Alkanoylgruppe gerad-und verzweigtkettig sein und an beliebiger Stelle der Kette ein- oder mehrfach substituiert sein.

Als Substituenten der Alkanoylgruppe sind Halogenatome, Ether-, Amino-, Hydroxyl- und Cyanogruppen geeignet. Bevorzugte Alkanoylgruppen sind Acetyl-, Propionoyl-, Butyryl-, Valeryl- und Caproylgruppen. Substituenten der als Rest $R_2$ infrage kommenden substituierten Benzoylgruppe sind Halogenatome, $C_{1-4}$-Alkyl-, Methoxy-, Amino-, Hydroxyl- und Cyanogruppen, wobei ein oder mehrere Substituenten an beliebigen Stellen der Benzoylgruppe stehen können.

Der Rest $R_2$ kann am Aromaten in der 2-, 3- oder 4-Stellung stehen.

Als mögliche Salze der Verbindungen der allgemeinen Formel I sind physiologisch verträgliche Salze von organischen oder anorganischen Säuren zu nennen. Als Salze sind besonders geeignet das Malonat, das Succinat, das Hydrochlorid und das Hydrobromid.

Die Verbindungen der allgemeinen Formel I sind Inhibitoren der Östrogenbiosynthese (Aromatasehemmer). Sie sind damit zur Behandlung von Krankheiten geeignet, die durch Östrogene bedingt oder von Östrogenen abhängig sind. So sind sie geeignet zur Behandlung von östrogeninduzierten oder -stimmulierten Tumoren,

wie zum Beispiel Mammakarzinom oder Prostatahyperplasie (The Lancet, 1984, 1237-1239).

Die erfindungsgemäßen Verbindungen sind auch wertvoll zur Beeinflussung der Fertilität. So kann eine männliche Infertilität, die aus erhöhten Östrogenspiegeln resultiert, mit den neuen Wirkstoffen behoben werden.

Ferner können die Verbindungen bei Frauen im fortpflanzungsfähigem Alter als Antifertilitätsmittel verwendet werden, um Ovolationen durch Östrogenentzug zu hemmen.

Wahrscheinlich sind Aromatasehemmer auch zur Behandlung des drohenden Herzinfarktes geeignet, da erhöhte Östrogenspiegel beim Mann einem Herzinfarkt vorangehen können (US-Patent 4,289,762).

Bekannte, eine die aromatasehemmende Wirkung aufweisende Substanzen sind neben Steroiden auch nichtsteroidale Substanzen; wie die zum Beispiel in den europäischen Patentanmeldungen, Veröffentlichungsnummern 0165777 bis 0165784 beschriebenen diversen Stickstoffheterocyclen, die in J. Med. Chem. 1986, 29, Seiten 1362-1369 beschriebenen substituierten Glutarsäureimide, die in der europäischen Patentanmeldung, Veröffentlichungsnummer 0165904, beschriebenen substituierten Imidazobenzole und die in der europäischen Patentanmeldung, Veröffentlichungsnummer 0236940, beschriebenen substituierten heterocyclisch substituierten Toluolnitrile.

Die Verbindungen der vorliegenden Anmeldung zeichnen sich gegenüber den bisher bekannten Verbindungen dadurch aus, daß sie das Enzymsystem der Aromatase stärker und zugleich selektiver hemmen. Die selektive Wirkung zeigt sich daran, daß andere Enzymsysteme in geringerem Umfang beeinträchtigt werden.

Die biologischen Teste A und B werden angewendet, um die Enzymaktivitäten und die Enzymselektivitäten der Verbindungen zu bestimmen.

Test A

Bestimmung der Aromataseaktivität

Die Fahigkeiten der Verbindungen, das Enzymsystem der Aromatase zu inhibieren, wird an Mikrosomen, erhalten aus Humanplacenta, getestet. Die Freisetzung von Tritium-markiertem Wasser ($^3H_2O$), das als Reaktionsprodukt bei der Aromatisierung des [1β-$^3$H]- Andostrendions zum Östron freigesetzt wird, wird nach der Methode von Thompson und Siiteri (J. Biol. Chem., 249, 5364-72 (1974)) gemessen. Die entsprechenden Hemmwerte ($K_i$, Aromatase) werden durch graphische Bestimmung, der Auftragung von 1/V gegen die Hemmkonzentration, gemäß der Methode von Dixon (Biochem. J. 94, 760 (1965) bestimmt.

Test B

Bestimmung der 11β-Hydroxylaseaktivität

Die 11β-Hydroxylaseaktivität wird an Mitochondrien, erhalten aus Rindernebennieren, gemessen. Die Umsetzung von [1,2-$^3$H]-17α-Hydroxy-11-desoxycorticosteron in Cortisol wird durch Dünnschichtchromatographie der entstehenden Reaktionsprodukte nach einer Methode von Mitani et al. (J. Biol. Chem., 250, 8010-15 (1975)) bestimmt. Es wird die Konzentration der untersuchten Verbindung bestimmt, bei der die Reaktion um 50% gehemmt wird, wobei die Substratkonzentration dem scheinbaren $K_{m'}$-Wert entspricht.

Beispielsweise für die erfindungsgemäßen Aromatasehemmer zeigen <4-[1-(1-Imidazol)-butyl]-phenyl>-pentyl-keton (Verbindung 2) und 4-[1-(1-Imidazolyl)-butyl]-benzoesäure-butylester (Verbindung 3) gegenüber dem literaturbekannten Aromatasehemmer (Verbindung 1) 4-[1-(1-Imidazolyl)-butyl]-benzonitril (Europäische Patentanmeldung, Veröffentlichungsnr. 0 236 940) selektivere Wirksamkeiten bei zugleich stärkeren Hemmwirkungen der Aromatase als Verbindung 1.

| | Test A | Test B |
|---|---|---|
| Verbindung | | $IC_{50'}$ |
| | $K_{i'}$ Aromatase | 11β-Hydroxylase |
| Verbindung 1 | 1,1 nmol/L | 94 nmol/L |
| Verbindung 2 | 0,42 nmol/L | 1,5 µmol/L |
| Verbindung 3 | 0,87 nmol/L | 750 nmol/L |

Die zu verabreichende Menge der Verbindungen schwanken innerhalb eines weiten Bereichs und können jede wirksame Menge abdecken. In Abhängigkeit des zu behandelnden Zustands und der Art der Verabreichung kann die Menge der verabreichten Verbindungen 0,0001-10 mg/kg Körpergewicht, vorzugsweise 0,001-1 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker,

Sorbit, Gelantine, Gleitmittel, Kieselsäure, Talkum usw., enthalten. Die einzelnen Dosierungseinheiten für die orale Applikation können beispielsweise 0,05-50 mg des Wirkstoffes (Aromataseinhibitors) enthalten.

Zur parenteralen Verabreichung können die Wirkstoffe in einem physiologisch verträglichen Verdünnungmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiergemisches verwendet. Als Beispiele für verwendete Öle sind zu nennen; Olivenöl, Erdnußöl, Baumwollöl, Sojabohnenöl, Rizinusöl und Sesamöl.

Die Verbindungen lassen sich auch in Form einer Depotinjektion oder eines Implantatpräparates anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silicone, wie zum Beispiel Siliconkautschuk. Die Wirkstoffe können außerdem zur perkutanen Aplikation zum Beispiel in Pflaster eingearbeitet werden.

Die Erfindung betrifft somit auch pharmazeutische Präparate und die Verwendung der Verbindungen zur Herstellung dieser Präparate zur Behandlung von östrogenbedingten Krankheiten.

Die Erfindung betrifft ferner Verfahren zur Herstellung von substituierten Imidazolen der allgemeinen Formel I gemäß Anspruch 1, dadurch gekennzeichnet, daß man

a) eine Verbindung der allgemeinen Formel II

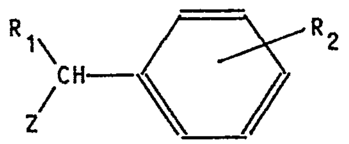

(II),

worin
$R_1$ und $R_2$ die in Anspruch 1 genannte Bedeutung haben und
Z eine Fluchtgruppe bedeutet,
mit einer Verbindung der allgemeinen Formel III

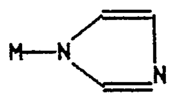

(III),

worin
M ein Wasserstoffatom oder ein Alkalimetallatom bedeutet, umsetzt oder

b) eine Verbindung der allgemeinen Formel IV

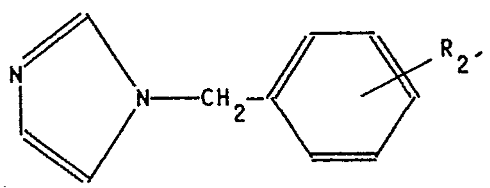

(IV),

worin
$R_{2'}$ dieselbe Bedeutung wie $R_2$ in Formel I hat mit der Maßgabe, daß $R_{2'}$ ein elektronenziehender, nicht deprotonierbarer Rest ist, in Gegenwart einer Base mit einer Verbindung der allgemeinen Formel V

$R_1$ - Z (V),

worin
$R_1$ die in Anspruch 1 genannte Bedeutung hat
und
Z eine Fluchtgruppe bedeutet,
umsetzt oder

c) eine Verbindung der allgemeinen Formel VI

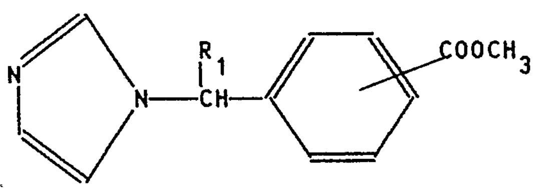

(VI),

worin

$R_1$ die in Anspruch 1 genannte Bedeutung hat,
mit einem Reduktionsmittel in die Hydroxymethylverbindung überführt, gegebenenfalls die Hydroxymethylverbindung mit einem Halogenierungsmittel zur Halogenmethylverbindung umsetzt, gegebenenfalls die Halogenmethylverbindung mit einem Metallcyanid in die Cyanomethylverbindung oder mit einem Metallalkoxid in die Alkoxymethylverbindung oder mit Ammoniak in die Aminomethylverbindung oder mit einem Amin zur substituierten Aminomethylverbindung, oder die Verbindung der allgemeinen Formel VI hydrolytisch in die Carboxylverbindung oder ins Carbonsäureamid oder unter Umesterungsbedingungen mit einem Alkohol in einen Carbonsäureester, gegebenenfalls die Carboxylverbindung mit einem Alkohol zum Carbonsäureester oder mit einem Halogenierungsmittel zum Carbonsäurehalogenid, gegebenenfalls das Carbonsäurehalogenid mit Ammoniak oder Aminen in Carbonsäureamide, gegebenenfalls die Hydroxymethylverbindung mit Braunstein in den Aldehyd, gegebenenfalls den Aldehyd mit Aminen oder substituierten Aminen in Imine oder substituierte Imine oder mit Wittigreagenzien in ungesättigte Verbindungen, gegebenenfalls die ungesättigten Verbindungen unter Hydrierungsbedingungen in gesättigte Verbindungen, gegebenenfalls den Aldehyd mit metallorganischen Verbindungen in die sekundären Alkohole und diese mit Oxidationsmittel in die substituierten Alkanoylderivate oder Benzoylderivate überführt.

Für die unter a) genannten Umsetzungen eignen sich als Fluchtgruppen Z literaturbekannte, leicht substituierbare Gruppen. Als solche zur Fluchtgruppenfähigkeit geeignete funktionelle Gruppen sind beispielsweise zu nennen die Mesyl-, Tosyl-, Triflat- und die Acetylgruppe. Als geeignet haben sich auch die Halogene Chlor und Brom erwiesen. Die Methoden sind u.a. in Eur. J. Med. Chem. 1979, Seiten 231-237, beschrieben. Als Substituenten M der allgemeinen Formel III kommen beispielsweise Wasserstoff- und Alkalimetallatome infrage. Als Alkalimetallatome sind Lithium, Natrium und Kalium bevorzugt.

Die Reaktionen, die unter a) aufgeführt sind, die Umsetzungen von Verbindungen der allgemeinen Formel II mit Verbindungen der allgemeinen Formel III, lassen sich in allen inerten organischen Lösungsmitteln durchführen. Als Lösungsmittel kommen beispielsweise Dimethylformamid, Dimethylsulfoxyd und diverse Ether (z.B. Tetrahydrofuran, Dioxan und Diethylether) infrage.
Die Umsetzung der Verbindungen der Formel II mit Imidazol (Formel III, M=H) kann auch ohne ein Lösungsmittel und bei Temperaturen zwischen dem Schmelzpunkt und dem Siedepunkt von Imidazol, vorzugsweise zwischen 100°C und 200°C, vorgenommen werden.
Die Herstellung der Verbindungen der allgemeinen Formel I kann auch gemäß Verfahren b) durchgeführt werden. Es erfolgt dabei Umsetzung von Verbindungen der allgemeinen Formel IV mit Verbindungen der allgemeinen Formel V. Als Fluchtgruppe Z der Verbindungen der allgemeinen Formel V sind alle gängigen Fluchtgruppen geeignet, z.B. die bei a) genannten.

Zur Herstellung der Verbindung der allgemeinen Formel I nach dem Verfahren b) wird mit Basen aus den Verbindungen der allgemeinen Formel IV das entsprechende Benzylanion erzeugt und mittels Standardmethoden mit den Verbindungen der allgemeinen Formel V umgesetzt. Die benzylische $-CH_2-$ Gruppe in den Verbindungen der allgemeinen Formel IV, die elektronenziehende, selbst nicht deprotonierbare Reste $R_{2'}$ enthalten müssen, läßt sich mittels Basen, z.B. durch Umsetzung mit tertiären Aminen, Natriumhydrid, Lithiumhydrid oder Lithiumdiisopropylamid deprotonieren und mit Verbindungen der allgemeinen Formel V zur Reaktion bringen.
Die erfindungsgemäßen Verbindungen der allgemeinen Formel I lassen sich auch nach dem Verfahren c) herstellen. Dabei werden Verbindungen der allgemeinen Formel VI nach Standardmethoden umgesetzt.

Die Carbonestergruppe der Verbindungen der allgemeinen Formel VI läßt sich durch Reduktionsmittel, beispielsweise Lithiumaluminiumhydrid, in die Hydroxymethylgruppe überführen. Diese wiederum läßt sich mit einem Halogenierungsmittel, beispielsweise Thionylchlorid, zur Halogenmethylverbindung, beispielsweise zum Chlormethylderivat, umsetzen. Der Halogensubstituent der Halogenmethylgruppe kann durch nucleophile Substitution, beispielsweise durch Umsetzung mit Natriumcyanid oder mit Natriummethylat oder mit Ammoniak oder Ethylamin, ausgetauscht werden. Es werden die entsprechenden Cyano-, Ethoxy-, Amino- oder Ethylaminomethylderivate erhalten.

Die Carbonestergruppe der Verbindungen der allgemeinen Formel VI läßt sich unter hydrolytischen Bedingungen, beispielsweise durch Reaktion mit wäßriger Säure, in die freie Carbonsäure bzw. unter aminolytischen Bedingungen, beispielsweise mit Ammoniaklösung, in das Carbonsäureamid überführen. Es ist aber auch möglich, die Carbonestergruppe unter Bedingungen einer Umesterungsreaktion mit Alkoholen in Gegenwart einer Säure, beispielsweise 4-Toluolsulfonsäure, in andere Ester umzuwandeln. Ebenso ist es möglich, Carbonsäureester über die Reaktionssequenz Carbonsäure und Carbonsäurehalogenid zu erhalten. Die als Zwischenprodukte erhältlichen Carbonsäurehalogenide können zur Herstellung von Carbonsäureamiden benutzt werden. Beispielsweise läßt sich ein Carbonsäurechlorid mit Ammoniak oder Aminen zum Carbonsäureamid oder substituierten Carbonsäureamid umsetzen.

Die bereits beschriebenen Hydroxymethylderivate lassen sich durch Oxidationsmittel, beispielsweise Mangandioxid (Braunstein), in die Formylderivate, die wiederum Ausgangsmaterialien der derivatisierten Formylderivate sind, umsetzen.

Die Formylderivate lassen sich darüber hinaus auch aus den Cyanoderivaten der Formel I durch Reduktion herstellen. Als Reduktionsmittel kommen komplexe Metallhydride, beispielsweise Natrium- oder Lithium-triäthoxyaluminiumhydrid oder Aluminium-Nickel-Legierungen in Säuren, vorzugsweise Ameisensäure, infrage.

Durch Reaktion der Formylderivate mit beispielsweise Aminogruppen enthaltenden Verbindungen, lassen

sich die Imine oder substituierten Imine erhalten. Es ist ebenso möglich, die Formylderivate mit Semicarbazid oder Derivaten des Semicarbazids in die Semicarbazone oder substituierten Semicarbazone, beispielsweise Thiosemicarbazone, zu überführen.

Die Formylderivate können auch benutzt werden, um zu Verbindungen zu gelangen, die als Rest $R_2$ einen substituierten Kohlenwasserstoffrest mit mehr als einem Kohlenstoffatom besitzen. Durch Umsetzung mit Wittigreagenzien lassen sich eine Reihe von Derivaten, beispielsweise die substituierten Zimtsäurederivate, erhalten. Die Doppelbindung im Reaktionsprodukt der Wittigreaktion läßt sich bei Bedarf hydrieren; so werden beispielsweise die substituierten Phenylpropionsäurederivate erhalten.

Die Formylderivate lassen sich weiter mit metallorganischen Reagenzien, beispielsweise Grignard-Reagenzien, in die entsprechenden sekundären Alkohole überführen. Diese sekundären Alkohole lassen sich mit Oxidationsmitteln, beispielsweise Mangandioxid (Braunstein), in die gegebenenfalls substituierten Alkanoyl- oder Benzoylderivate überführen.

## Beispiele

### Beispiel 1

4-[1-(1-Imidazolyl)-butyl]-benzoesäuremethylester

6 g 4-Formylbenzoesäure werden in 100 ml Tetrahydrofuran gelöst und mit 100 ml einer 1-molaren Lösung von Propylmagnesiumbromid in Tetrahydrofuran versetzt. Nach 6 Stunden Reaktionszeit bei Raumtemperatur wird das Gemisch in 100 ml 2 M Salzsäure eingerührt, mit Ether extrahiert und die Etherphase eingedampft. Das kristalline Material wird mit Toluol über eine Nutsche abfiltriert und in 45 ml Dioxan und 5 ml Wasser gelöst und mit 1,5 g Natriumborhydrid behandelt. Nach 15 Minuten bei Raumtemperatur wird das Gemisch in 1 M Salzsäure gegeben, mit Essigester extrahiert, getrocknet, eingedampft und an 120 g Kieselgel chromatographiert [Toluol/ Essigsäure/Wasser (10:10:1; Oberphase nach Zusatz von wenig Wasser)]. Es werden 4,04 g 4-(1-Hydroxybutyl)-benzoesäure vom Schmelzpunkt 109 -113 °C erhalten.

500 mg davon werden mit 2,5 Thionylchlorid 2,5 Stunden bei Raumtemperatur gerührt. Nach Abdestillieren des Thionylchlorids werden 2 ml Methanol und 1 ml Pyridin zugesetzt, 20 Stunden bei Raumtemperatur gerührt und anschließend das Gemisch in 10 ml 1 M Salzsäure + 10 ml Ether verteilt. Die Etherphase wird getrocknet und eingedampft, der ölige Rückstand von 4-(1-Chlorbutyl)-benzoesäuremethylester wird in 2 ml Dimethylformamid gelöst und zu einer aus 270 mg Imidazol und 120 mg 80 %iger Natriumhydrid-Ölsuspension in 3 ml Dimethylformamid bereiteten Lösung gegeben. Nach 20 Stunden Rühren bei Raumtemperatur wird auf überschüssige 1 M Salzsäure gegeben und mit Ether extrahiert. Die Wasserphase wird mit festem Kaliumcarbonat alkalisiert und mit Essigester extrahiert. Nach Trocknen und Eindampfen des Lösungsmittels wird bei 210 - 230 °C/0,03 mbar am Kugelrohr destilliert. Man erhält 264 mg 4-[1-(1-Imidazolyl)-butyl]-benzoesäuremethylester.

### Beispiel 2

a) 4-[1-(1-Imidazolyl)-butyl]-benzoesäure

23 g des Esters aus Beispiel 1 werden in 200 ml konzentrierter Salzsäure 20 Stunden unter Rückfluß erhitzt. Nach Eindampfen im Vakuum wird der Rückstand in Wasser gelöst und mit 50 %iger Natronlauge auf pH 8 gebracht. Danach wird mit 1 M Salzsäure auf pH 5-6 eingestellt und zur Trockne eingeengt. Der kristalline Rückstand wird mehrfach mit Essigester ausgekocht und filtriert. Aus dem Filtrat erhält man nach Einengen 20 g 4-[1-(1-Imidazolyl)-butyl]-benzoesäure vom Schmelzpunkt 171 - 172 °C.

b) 4-[1-(1-Imidazolyl)-butyl]-benzoesäure, Hydrochlorid

1,0 g 4-[1-(1-Imidazolyl)-butyl]-benzoesäure werden in 20 ml Ethanol gelöst und mit 10 ml konzentrierter Salzsäure versetzt. Das Reaktionsgemisch wird in 100 ml Wasser eingefällt und der ausgefallene Niederschlag abgesaugt. Nach Umkristallisation aus Ethanol werden 0,95 g 4-[1-(1-Imidazolyl)-butyl]-benzoesäure, Hydrochlorid vom Schmelzpunkt 38 °C erhalten.

### Beispiel 3

4-[1-(1-Imidazolyl)-butyl]-benzamid

5 g der Säure aus Beispiel 2 werden mit 20 ml Thionylchlorid 3 Stunden bei Raumtemperatur gerührt. Nach Eindampfen des überschüssigen Thionylchlorids erhält man das Hydrochlorid des Säurechlorids als zähes Öl.

2,5 g des Säurechlorids werden in 20 ml Methylenchlorid gelöst. Man leitet 10 Minuten Ammoniak ein, zentrifugiert, engt den klaren Überstand ein und chromatographiert an Kieselgel. Mit Methylenchlorid/Methanol (97:3) werden 170 mg 4-[1-(1-Imidazolyl)-butyl]-benzamid vom Schmelzpunkt: 132 °C erhalten.

Beispiel 4

4-[1-(1-Imidazolyl)-butyl]-benzonitril

a) 4,9 g des Amids aus Beispiel 3 werden in 50 ml Dioxan und 3,23 ml Pyridin gelöst und unter Eiskühlung bei 5 °C Innentemperatur mit 3,16 ml Trifluoressigsäureanhydrid versetzt. Nach 20 Stunden rühren bei Raumtemperatur wird auf halbegesättigte Kaliumcarbonatlösung gegeben, mit Ether extrahiert und die Etherphase getrocknet und eingeengt. Nach Destillation am Kugelrohr bei 200-215 °C/0,05 mbar erhält man 3,2 g 4-[1-(1-Imidazolyl)-butyl]-benzonitril, das nach Kristallisation aus Ether bei 63-71 °C schmilzt.

b) Durch Umsetzung von 4-Brombenzaldehyd mit Propylmagnesiumbromid unter Standardbedingungen wird das 1-(4-Bromphenyl)-1-butanol, mit dem Siedepunkt 180 °C/0,03 mbar, erhalten.

380 g 1-(4-Bromphenyl)-1-butanol, werden in einem Kolben vorgelegt und bei -30 °C mit 240 ml Thionylchlorid versetzt und eine Stunde bei Raumtemperatur nachgerührt. Das Gemisch wird über Nach stehen gelassen. Das Reaktionsgemisch wird in 10 l Wasser eingerührt und anschließend mit Ether extrahiert. Die Etherphasen werden getrocknet und im Vakuum eingeengt. In einem zweiten Kolben werden 123 g Imidazol vorgelegt und in 1500 ml Dimethylformamid gelöst. Unter Stickstoff werden nun 58 g Natriumhydrid langsam zugegeben. Es wird 2 Stunden bei Raumtemperatur nachgerührt und die erhaltene Chlorverbindung langsam zugetropft. Das Reaktionsgemisch wird über Nacht stehen gelassen. Danach wird das Reaktionsgemisch in 10 l Wasser eingefällt, 3x mit jeweils 1 l Ether extrahiert und die organische Phase mit insgesamt 5 l Wasser nachgewaschen. Anschließend wird im Vakuum das restliche Lösungsmittel abgezogen und der verbleibende Rückstand fraktioniert destilliert. Es werden 300 g 1-[1-(4-Bromphenyl)-butyl]-imidazol vom Siedepunkt 200 °C/0,025 mbar erhalten.

2,79 g 1-[1-(4-Bromphenyl)-butyl]-imidazol und 1,8 g Kupfer-I-Cyanid werden in 5 ml N-Methylpyrrolidon 4 Stunden bei 180 °C unter einer Schutzgasatmosphäre (Argon) erhitzt. Nach dem Abkühlen wird in 50 ml Amoniaklösung eingefällt, 15 Minuten bei Raumtemperatur nachgerührt und mit Essigester extrahiert. Nach dem Einengen im Vakuum werden 1,95 g dunkles Öl erhalten. Dieses Öl wird am Kugelrohr im Vakuum distilliert; Siedepunkt 200-215 °C/0,05 mbar. Das erhaltene Destillat wird aus Ethanol/ Petrolether umkristallisiert. Man erhält 1,54 g 4-[1-(1-Imidazolyl)-butyl]-benzonitril vom Schmelzpunkt 63-71 °C.

Beispiel 5

4-[1-(1-Imidazolyl)-butyl]-benzylalkohol

1,0 g Benzoesäuremethylester aus Beispiel 1 werden in 15 ml Tetrahydrofuran gelöst und mit 0,15 g (3,9 mmol) Lithiumaluminiumhydrid versetzt und 1 Stunde bei Raumtemperatur belassen. Die Reaktionslösung wird mit 50 %iger Natronlauge versetzt und die organische Phase abdekantiert. Die organische Phase wird im Vakuum eingeengt und am Kugelrohr destilliert, Siedepunkt 200 °C/ 0,03 mbar. Es werden 0,6 g 4-[1-(1-Imidazolyl)-butyl]-benzylalkohol erhalten.

Beispiel 6

4-[1-(1-Imidazolyl)-butyl]-benzaldehyd

a) 0,1 g des substituierten Benzylalkohols aus Beispiel 5 werden in 10 ml Methylenchlorid gelöst, mit 0,15 g Mangandioxyd versetzt und 6 Stunden am Rückfluß gekocht. Nach dem Abkühlen wird die Reaktionsmischung über Kieselgur filtriert und im Vakuum eingeengt. Der Rückstand wird am Kugelrohr destilliert, Siedepunkt 200 °C/0,03 mbar. Es werden 0,08 g 4-[1-(1-Imidazolyl)-butyl]-benzaldehyd erhalten.

b) 10 g Nitril aus Beispiel 4 werden mit 10 g Aluminium-Nickel-Legierung in 150 ml Ameisensäure/Wasser (75:25) 1 Stunde unter Rückfluß gekocht. Man filtriert über Kieselgur, alkalisiert das Filtrat mit festem Kaliumcarbonat, gibt 100 ml wässrigen Ammoniak hinzu und extrahiert mit Ether. Nach Trocknen und Eindampfen des Ethers wird am Kugelrohr destilliert; man erhält 9,1 g 4-[1-(1-Imidazolyl)-butyl]-benzaldehyd, Siedepunkt 200 °C/0,03 mbar.

Beispiel 7

1-[4-[1-(1-Imidazolyl)-butyl]-phenyl]-ethanol

460 mg des Aldehyds aus Beispiel 6 in 8 ml Ether werden mit 1,5 ml einer 3 M Methylmagnesiumbromidlösung in Ether bei Eiskühlung versetzt und 20 Stunden bei Raumtemperatur gerührt; es wird 10 ml Tetrahydrofuran zugesetzt und 6 Stunden auf 60 °C erwärmt. Man gibt auf 1 M Salzsäure, extrahiert mit Ether, alkalisiert dann die Wasserphase mit Kaliumcarbonat und extrahiert mit Ether. Nach Trocknen und Eindampfen dieser Etherphase wird der Rückstand an Kieselgel chromatographiert (Methylenchlorid/Methanol (95:5)). Erhalten werden 300 mg der Titelverbindung.

Beispiel 8

4′-[1-(1-Imidazolyl)-butyl]-acetophenon

200 mg des Alkohols aus Beispiel 7 werden in 10 ml Methylenchlorid mit 1 g Braunstein über Nacht gerührt. Man saugt ab, dampft ein und destilliert den Rückstand bei 180 °C/0,03 hPa. Man erhält 100 mg der Titelverbindung als farbloses Öl.

Beispiel 9

1-[4-[1-(1-Imidazolyl)-butyl]-phenyl]-2-methyl-1-propanol
Analog Beispiel 7 unter Verwendung von 2-Propylmagnesiumbromid.

Beispiel 10

[4-[1-(1-Imidazolyl)-butyl]-phenyl]-2-propylketon
Analog Beispiel 8 aus der Verbindung des Beispiel 9; Siedepunkt 250 °C/0,04 mbar.

Beispiel 11

4′-[1-(1-Imidazolyl)-butyl)-propiophenon

Das gemäß Beispiel 4 erhaltene 4-[1-(1-Imidazolyl)-butyl]-benzonitril wird unter Standardbedingungen mit Ethylmagnesiumbromid umgesetzt. Es werden 0,65 g 4-[1-(1-Imidazolyl)-butyl)-propiophenon vom Siedepunkt 235 °C/0,03 mbar erhalten.

Analog Beispiel 11 werden durch Umsetzung von 4-[1-(1-Imidazolyl)-butyl]-benzonitril mit den entsprechenden Grignardverbindungen die Verbindungen der Beispiele 12-16 erhalten.

Beispiel 12

[4-[1-(1-Imidazolyl)-butyl]-phenyl-propylketon
Siedepunkt 200 °C/0,03 mbar.

Beispiel 13

[4-[1-(1-Imidazolyl)-butyl]-phenyl]-butylketon
Siedepunkt 200 °C/0,03 mbar.

Beispiel 14

[4-[1-(1-Imidazolyl)-butyl]-phenyl]-pentylketon
Siedepunkt 210 °C/0,04 mbar.

Beispiel 15

[4-[1-(1-Imidazolyl)-butyl]-phenyl]-hexylketon
Siedepunkt 250 °C/0,03 mbar.

Beispiel 16

Cyclohexyl-[4-[1-(1-Imidazolyl)-butyl]-phenyl]-keton
Siedepunkt 250 °C/0,04 mbar.

Beispiel 17

4-[1-(1-Imidazolyl)-butyl]-benzophenon

Das 4-[1-(1-Imidazolyl)-butyl]-benzonitril wird mit Phenylmagnesiumbromid unter Standardbedingungen analog Beispiel 11 in Tetrahydrofuran umgesetzt. Es werden 0,83 g 4-[1-(1-Imidazolyl)-butyl]-benzophenon vom Siedepunkt 175 °C/0,03 mbar erhalten.

Beispiel 18

4′-[Cyclohexyl-(1-imidazolyl)-methyl]-acetophenon

Aus 10 g 4′-Bromacetophenon-dimethylketal und 2 g Magnesium in 100 ml Ether wird unter Verwendung von 1,2-Dibromethan eine Grignard-Lösung hergestellt. Dazu wird die Lösung von 4 g Cyclohexanaldehyd in 20 ml Ether getropft. Nach 20 Stunden bei Raumtemperatur werden 100 ml 1 M Salzsäure zugegeben und wird 1 Stunde kräftig gerührt. Die Etherphase wird abgetrennt, getrocknet, eingedampft und der Rückstand an

Kieselgel mit Toluol/Essigester (Gradient 0-20 % Essigester) chromatographiert. Man erhält 5 g (4-Acetylphenyl)-cyclohexylmethanol.

Dieses wird mit 10 ml Thionylchlorid 1 Stunde bei Raumtemperatur belassen. Das überschüssige Thionylchlorid wird im Vakuum abgezogen und der Rückstand mit 10 g Imidazol 20 Stunden bei 130 °C zur Reaktion gebracht. Man gießt nach Erkalten auf einen Überschuß von 2 M Salzsäure, extrahiert mit Ether, alkalisiert die wäßrige Phase mit Kaliumcarbonat und extrahiert mit Essigester. Nach Trocknen und Eindampfen des Lösemittels wird am Kugelrohr destilliert. Erhalten werden 2 g der Titelverbindung, Siedepunkt 230-250 °C/0,03 mbar.

Beispiel 19

4-[1-(1-Imidazolyl)-butyl]-benzoesäuredimethylamid

1,2 g 4-[1-(1-Imidazolyl)-butyl]-benzoesäure werden in 10 ml Dimethylformamid gelöst, bei 0 °C mit 1 ml N-Ethylmorpholin und 1 ml Chlorameisensäureisobutylester versetzt. Es wird 30 Minuten bei 0 °C gerührt, 2 ml einer Dimethylaminlösung zugetropft und 2 Stunden bei 0 °C gerührt. Es wird in 500 ml Wasser eingerührt, mit Essigester extrahiert und die organische Phase im Vakuum eingeengt. Der verbleibende Rückstand wird am Kugelrohr destilliert, Siedepunkt: 260 °C/0,03 mbar. Es werden 0,9 g 4-[1-(1-Imidazolyl)-butyl]-benzoesäuredimethylamid erhalten.

Beispiel 20

4-[1-(1-Imidazolyl)-butyl]-benzoesäurepyrrolidid

5,0 g 4-[1-(1-Imidazolyl)-butyl]-benzoesäure (aus Beispiel 2) werden 2 Stunden mit 50 ml Thionylchlorid bei Raumtemperatur gerührt. Anschließend werden 100 ml Toluol zugegeben und die Lösung im Vakuum eingeengt. Es wird in 40 ml Methylenchlorid gelöst, mit 5 ml Pyrrolidin versetzt und 3 Stunden bei Raumtemperatur gerührt. Dazu werden 50 ml Wasser gegeben und mit Essigester extrahiert. Es wird erneut im Vakuum eingeengt und der Rückstand am Kugelrohr destilliert; Siedepunkt 260 °C/0,02 mbar. Es werden 4-[1-(1-Imidazolyl)-butyl]-benzoesäurepyrrolidid erhalten.

Analog Beispiel 20 werden durch Umsetzungen von 4-[1-(1-Imidazolyl)-butyl]-benzoesäure über das Säurechlorid mit den entsprechenden Aminen die Verbindungen der Beispiele 21-30 erhalten.

Beispiel 21

N-Butyl-4-[1-(1-imidazolyl)-butyl]-benzamid
Siedepunkt 250° C/0,04 mbar.

Beispiel 22

N-Cyclohexyl-4-[1-(1-imidazolyl)-butyl]-benzamid
Siedepunkt 250° C/0,03 mbar.

Beispiel 23

4-[1-(1-Imidazolyl)-butyl]-benzanilid
Schmelzpunkt: 173-175° C

Beispiel 24

N-(4′-Tolyl)-4-[1-(1-Imidazolyl)-butyl]-benzamid
Schmelzpunkt 155-157° C.

Beispiel 25

N-(4-Chlophenyl)-4-[1-(1-imidazolyl)-butyl]-benzamid
Schmelzpunkt 152° C.

Beispiel 26

N-Benzyl-4-[1-(1-imidazolyl)-butyl]-benzamid
Schmelzpunkt 137-140° C.

Beispiel 27

N-(3-Pyridyl)-4-[1-(1-Imidazolyl)-butyl]-benzamid

Beispiel 28

4-[1-(1-Imidazolyl)-butyl]-benzoesäure-(4-methylpiperidid)
Siedepunkt 250° C/0,03 mbar.

Beispiel 29

4-[1-(1-Imidazolyl)-butyl]-N-methyl-N-phenyl-benzamid

Beispiel 30

4-[1-(1-Imidazolyl)-butyl]-benzoesäure (N′-methylpiperazid)
Siedepunkt 200° C/0,030 mbar.

Beispiel 31

4-[1-(1-Imidazolyl)-butyl]-benzoesäuremethylester

1,0 g 4-[1-(1-Imidazolyl)-butyl]-benzoesäure, Hydrochlorid (aus Beispiel 2) werden in 30 ml Methanol gelöst, 10 Minuten Chlorwasserstoffgas eingeleitet, 30 Minuten am Rückfluß gekocht und eingeengt. Der Rückstand wird am Kugelrohr destilliert, Siedepunkt 190 ° C/0,03 mbar. Es werden 0,55 g 4-[1-(1-Imidazolyl)-butyl]-benzoesäuremethylester erhalten.

Beispiel 32

4-[1-(1-Imidazolyl)-butyl]-benzoesäurebutylester

Analog Beispiel 31 wird durch Umsetzung der substitituierten Benzoesäure mit Butanol der 4-[1-(1-Imidazolyl)-butyl]-benzoesäurebutylester vom Siedepunkt 190° C/0,03 mbar erhalten.

Beispiel 33

4-[1-(1-Imidazolyl)-butyl]-benzoesäure-isopropylester
Siedepunkt 200° C/0,03 mbar.

Beispiel 34

4-[1-(1-Imidazolyl)-butyl]-benzoesäure-(1-methylbutyl)-ester
Siedepunkt 200° C/0,03 mbar, analog Beispiel 31.

Beispiel 35

4-[1-(1-Imidazolyl)-butyl]-benzoesäure-cyclohexylester

1 g 4-[1-(1-Imidazolyl)-butyl]-benzoesäure werden analog Beispiel 20 in das Säurechlorid überführt; dieses wird in 10 ml Methylenchlorid gelöst, mit 360 mg Cyclohexanol und 3 ml Pyridin versetzt, nach 20 Stunden bei Raumtemperatur auf überschüssige 1 M Salzsäure gegeben und mit Ether extrahiert. Die Wasserphase wird mit Kaliumcarbonat alkalisiert und mit Essigester extrahiert. Nach Trocknen und Einengen wird der Rückstand am Kugelrohr destilliert. Man erhält 915 mg der Titelverbindung als Öl vom Siedepunkt 200° C/0,03 mbar.

Analog Beispiel 35 werden durch Umsetzung von 4-[1-(1-Imidazolyl)-butyl]-benzoesäure über das Säurechlorid mit den entsprechenden Alkoholen die Verbindungen der Beispiele 36-38 erhalten.

Beispiel 36

4-[1-(1-Imidazolyl)-butyl]-benzoesäure-(2-methoxyethyl)-ester
Siedepunkt 250 ° C/0,03 mbar.

Beispiel 37

4-[1-(1-Imidazolyl)-butyl]-benzoesäure-(2-methylmercaptoethyl)-ester
Siedepunkt 200 ° C/0,03 mbar.

Beispiel 38

4-[1-(1-Imidazolyl)-butyl]-benzoesäure-(2-dimethylaminoethyl)-ester
Siedepunkt 220° C/0,03 mbar.

Beispiel 39

4-[1-(1-Imidazolyl)-butyl]-benzoesäure-tert.-butylester
1 g 4-[1-(1-Imidazolyl)-butyl]-benzoesäure werden mit 3,4 g N,N-Dimethylformamid-di-tert.-butylacetal in 6 ml Toluol 1 Stunde auf 100 °C erwärmt. Dann wird in Ether/1 M Salzsäure verteilt, die Wasserphase mit Kaliumcarbonat alkalisiert und mit Essigester extrahiert; nach Eindampfen des Lösemittels wird am Kugelrohr destilliert. Man erhält 0,58 g der Titelverbindung, Siedepunkt 200 °C/0,03 mbar.

Beispiel 40

1-[4-[1-(1-Imidazolyl)-butyl]-phenyl]-1-propanol
0,45 g 4-[1-(1-Imidazolyl)-butyl]-propiophenon aus Beispiel 11 werden in 10 ml Dioxan gelöst und mit 0,130 g Natriumborhydrid versetzt und über Nacht bei Raumtemperatur gerührt. Der Ansatz wird anschließend in 100 ml Wasser eingefällt und mit Ether extrahiert. Das Lösungsmittel wird in Vakuum eingeengt und der verbleibende Rückstand bei 220 °C/0,03 mbar am Kugelrohr destilliert. Es werden 0,250 g 1-[4-[1-(1-Imidazolyl)-butyl]-phenyl]-1-propanol erhalten.

Beispiel 41

3′-[1-(1-Imidazolyl)-butyl)-trans-zimtsäureethylester

a) 1-[1-(3-Bromphenyl)-butyl]-imidazol
3-Brombenzaldehyd werden mit Propylmagnesiumbromid unter Standardbedingungen zum 1-(3-Bromphenyl)-butanol umgesetzt.
4,0 g (17,5 mmol) 1-(3-Bromphenyl)-butanol werden mit 4 ml Thionylchlorid 1 Stunde bei Raumtemperatur gerührt. Zum Reaktionsgemisch werden 10 ml Toluol gegeben. Nach Einengen im Vakuum erhält man 1-Chlor-1-(3-bromphenyl)-butanol. Es werden 1,3 g Imidazol in 20 ml Dimethyformamid gelöst und unter Argon-Schutzgasatmosphäre mit 0,6 g Natriumhydrid (80 %ig in Öl) versetzt. Es wird 1 Stunde bei Raumtemperatur gerührt. Dazu tropft man die Chlorverbindung in 15 ml Dimethylformamid und läßt über Nacht stehen. Die Mischung wird in 200 ml Wasser eingefällt und mit Essigester extrahiert. Die organische Phase wird im Vakuum eingeengt und anschließend am Kugelrohr bei 150° C/0,03 mbar destilliert. Es werden 1,6 g 1-[1-(3-Bromphenyl)-butyl]-imidazol als viskoses Öl erhalten.
b) 11,0 g der Verbindung a), 8,8 ml Acrylsäureethylester, 20 ml Tributylamin, 1,86 g Triphenylphosphin und 0,82 g Palladium-II-acetat werden zusammengegeben und 18 Stunden bei 100 °C gerührt (J. Am. Chem. Soc. 96, 1133 (1974)). Danach wird das Reaktionsgemisch in 200 ml 1 n Salzsäure eingefällt. Es wird 3x mit Ether extrahiert und der Extrakt verworfen. Die wässrige Lösung wird mit 50 %iger Natronlauge alkalisch gestellt und erneut mit Ether extrahiert. Die organischen Phasen werden vereinigt, getrocknet und eingeengt. Der verbleibende Rückstand wird am Kugelrohr destilliert, Siedepunkt 230 °C/0,03 mbar. Es werden 7,63 g 3′-[1-(1-Imidazoloyl)-butyl]-trans-zimtsäureethylester (b)) erhalten.

Beispiel 42

3′-[1-(1-Imidazolyl)-butyl]-trans-zimtsäure
7,6 g 3′-[1-(1-Imidazolyl)-butyl]-trans-zimtsäureethylester (aus Beispiel 41) werden in 100 ml Ethanol gelöst und mit 50 ml 10 %iger Natronlauge versetzt. Das Reaktionsgemisch wird 2 Stunden bei 100 °C stehen gelassen. Es wird zur Trockne eingeengt, der Rückstand mit konzentrierter Salzsäure sauer gestellt, das ausgefallene Salz abgesaugt und mit Ethanol gespült. Es werden 5,26 g (67 % d. Th.) 3′-[1-(1-Imidazolyl)-butyl]-trans-zimtsäure erhalten.

Beispiel 43

3-[3-[1-(1-Imidazolyl)-butyl]-phenyl]-propionsäure
1,08 g 3-[1-(1-Imidazolyl)-butyl]-zimtsäure (Beispiel 42) werden in 20 ml Methanol gelöst und mit 100 mg Palladium auf Kohle (10 %) versetzt und bei Normaldruck hydriert. Nach 2 Stunden nimmt das Reaktionsgemisch keinen Wasserstoff mehr auf. Danach wird der Katalysator über Kieselgur abfiltriert und das Filtrat im Vakuum eingeengt. Es werden 1,05 g an 3-[3-(1-Imidazolyl)-butyl]-phenyl]-propionsäure erhalten.

Beispiel 44

4-[1-(1-Imidazolyl)-butyl]-benzylchlorid, Hydrochlorid

Durch Reaktion des entsprechend substituierten Benzylalkohols aus Beispiel 5 mit Thionylchlorid unter Standardbedingungen wird das 4-[1-(1-Imidazolyl)-butyl]-benzylchlorid, Hydrochlorid erhalten.

Beispiel 45

1-[1-(4-Methoxymethylphenyl)-butyl]-imidazol

0,4 g substituiertes Benzylchlorid aus Beispiel 44 werden in 10 ml Methanol gelöst und tropfenweise mit einer methanolischen Lösung, bereitet aus 20 ml Methanol und 0,42 g Natriumhydrid, versetzt. Es wird über Nacht bei Raumtemperatur gerührt. Anschließend wird das Methanol im Vakuum abgezogen, der Rückstand mit 50 ml Wasser versetzt und mit Ether extrahiert. Die organische Phase wird getrocknet und eingeengt. Es wird am Kugelrohr destilliert, Siedepunkt 250 °C/0,03 mbar. Es werden 0,19 g an 1-[1-(4-Methoxymethylphenyl)-butyl]-imidazol erhalten.

Beispiel 46

1-[1-(4-Butoxymethylphenyl)-butyl]-imidazol

Analog Beispiel 45 wird durch Umsetzung des substituierten Benzylchlorids mit Natriumbutylat das 1-[1-(4-Butoxymethylphenyl)-butyl]-imidazol erhalten. Siedepunkt 250° C/0,03 mbar

Beispiel 47

1-[1-(4-Phenoxymethylphenyl)-butyl]-imidazol

Siedepunkt 250° C/0,03 mbar

Analog Beispiel 45 wird durch Umsetzung des substituierten Benzylchlorids mit Natriumphenolat das 1-[1-(4-Butoxymethylphenyl)-butyl]-imidazol erhalten.

Beispiel 48

4-[1-(1-Imidazolyl)-butyl]-phenylacetonitril

0,545 g des aus Beispiel 44 erhaltenen Benzylchlorids werden in 10 ml Dimethylsulfoxyd gelöst und mit 0,125 g Natriumcyanid versetzt und über Nacht bei Raumtemperatur gerührt. Die Reaktionsmischung wird in 200 ml Wasser eingefällt und mit Essigester extrahiert. Die organische Phase wird im Vakuum zur Trockne eingeengt und der Rückstand am Kugelrohr destilliert, Siedepunkt 250 °C/0,03 mbar. Es werden 0,4 g 4-[1-(1-Imidazolyl)-butyl]-phenylacetonitril erhalten.

Beispiel 49

1-[4-[1-(1-Imidazolyl)-butyl]-benzyl]-pyrrolidin

0,15 g des substituierte Benzoesäurepyrrolidid (Beispiel 20) werden in 3 ml Tetrahydrofuran gelöst und zu einer Suspension bestehend aus 0,2 g Lithiumaluminiumhydrid in 5 ml Tetrahydrofuran, getropft. Es wird 2 Stunden am Rückfluß gekocht. Die Reaktionslösung wird mit 50%iger Natronlauge versetzt und die organische Phase abdekantiert und zur Trockne eingeengt. Der Rückstand wird am Kugelrohr destilliert, Siedepunkt 210° C/0,03 mbar. Es werden 0,1 g an 1-[4-[1-(1-Imidazolyl)-butyl]-benzyl]-pyrrolidin erhalten.

Beispiel 50

1-[1-(4-Hydroxyiminomethylphenyl)-butyl]-imidazol

0,5 g 4-[1-(1-Imidazolyl)-butyl]-benzaldehyd (siehe Beispiel 6) werden in 2 ml Pyridin gelöst und mit 0,215 g Hydroxylaminhydrochlorid versetzt. Es wird über Nacht bei Raumtemperatur gerührt und in 30 ml Wasser eingefällt und mit Ether extrahiert. Nach dem Entfernen des Lösungsmittels unter Vakuum werden 0,4 g 1-[1-(4-Hydroxyiminomethylphenyl)-butyl]-imidazol als gelbes Öl erhalten.

Beispiel 51

1-[1-(4-Methoxyiminomethylphenyl)-butyl]-imidazol

0,5 g 4-[1-(1-Imidazolyl)-butyl]-benzaldehyd (aus Beispiel 6) werden mit 0,42 g O-Methylhydroxylamin-Hydrochlorid in 20 ml Pyridin gelöst und über Nacht gerührt. Es wird in 100 ml Wasser eingegossen und mit Essigester extrahiert. Die organische Phase wird im Vakuum eingeengt und am Kugelrohr destilliert, Siedepunkt 200 °C/0,03 mbar. Es werden 0,39 g 1-[1-(4-Methoxyiminomethylphenyl)-butyl]-imidazol erhalten.

Analog Beispiel 50 und 51 werden durch Umsetzungen von 4-[1-(1-Imidazolyl)-butyl]-benzaldehyd mit den entsprechenden Aminen die Verbindungen der Beispiele 52-56 erhalten.

Beispiel 52

1-[1-(4-Ethoxyiminomethyl-phenyl)-butyl]-imidazol

Beispiel 53

1-[1-(4-Allyloxyiminomethyl-phenyl)-butyl]-imidazol

Beispiel 54

1-[1-(4-Benzyloxyiminomethyl-phenyl)-butyl]-imidazol

Beispiel 55

1-[1-[4-(4-Nitrobenzyloxyimino-methyl)-phenyl)-butyl]-imidazol

Beispiel 56

1-[1-[4-(2,3,4,5,6-Pentafluorbenzyloxyimino-methyl)-phenyl]-butyl]-imidazol

Beispiel 57

α-[4-[1-(1-Imidazolyl)-butyl]-phenyl]-benzylalkohol
Durch Reduktion des Ketons aus Beispiel 17 mit Natriumborhydrid in Dioxan/Wasser (90:10); Siedepunkt 250 °C/0,03 mbar.

Beispiel 58

1-[1-(4-Semicarbazonomethylphenyl)-butyl]-imidazol
0,5 g 4-[1-(1-Imidazolyl)-butyl]-benzaldehyd (Beispiel 6) werden in 20 ml Pyridin gelöst und mit 1,37 g Semicarbazin-Hydrochlorid versetzt. Es wird über Nacht bei Raumtemperatur gerührt, die Reaktionsmischung anschließend in 100 ml Wasser eingefällt und mit Methylenchlorid extrahiert. Die organische Lösung wird im Vakuum eingeengt. Erhalten werden 0,42 mg der Titelverbindung vom Schmelzpunkt 38°C.

Beispiel 59

4-[1-(1-Imidazolyl)-butyl]-trans-zimtsäureethylester
0,228 g 4-[1-(1-Imidazolyl)-butyl]-benzaldehyd (Beispiel 6) werden in 5 ml Dioxan gelöst und mit 0,35 g Carbethoxymethylentriphenylphosphoran versetzt und 48 Stunden bei Raumtemperatur gerührt. Die Reaktionsmischung wird in 100 ml Wasser eingegossen und mit Essigester extrahiert. Die organischen Phasen werden vereinigt und zur Trockne eingeengt, der Rückstand am Kugelrohr destilliert. Erhalten werden 0,15 g der Titelverbindung mit Siedepunkt 200-220°C/0,03 mbar.

Beispiel 60

4-[1-(1-Imidazolyl)-butyl]-trans-zimtsäure, Hydrochlorid
Durch Verseifung des Ethylesters aus Beispiel 59 und anschließende Überführung in das Hydrochlorid wird die Titelverbindung erhalten.

Beispiel 61

3-[1-(1-Imidazolyl)-butyl]-propionsäureethylester
Durch Veresterung der Säure aus Beispiel 43 mit Ethanol unter Standardbedingungen wird die Titelverbindung erhalten.

**Patentansprüche**

1. N-Substituierte Imidazole der allgemeinen Formel I

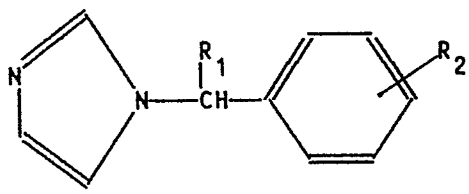

(I),

worin

$R_1$ ein Wasserstoffatom, einen gesättigten oder ungesättigten, gerad- oder verzweigtkettigen Kohlenwasserstoffrest mit 1 bis 10 Kohlenstoffatomen, einen cyclischen Kohlenwasserstoffrest mit 3 bis 9 Kohlenstoffatomen, einen Cycloalkylalkylrest mit 4 bis 12 Kohlenstoffatomen oder einen Arylalkylrest mit 7 bis 10 Kohlenstoffatomen,

und

$R_2$ einen substituierten gerad- oder verzweigtkettigen, gesättigten oder ungesättigten Kohlenwasserstoffrest mit 1 bis 10 Kohlenstoffatomen, eine gegebenenfalls derivatisierte Formylgruppe, eine gegebenenfalls substituierte Alkanoylgruppe mit 2 bis 10 Kohlenstoffatomen, eine gegebenenfalls substituierte Benzoylgruppe oder eine gegebenenfalls derivatisierte Carboxylgruppe

bedeutet,

sowie die Verbindungen in Form ihrer Salze.

2. Verbindungen gemäß Anspruch 1

4-(1-(1-Imidazolyl)-butyl)-benzoesäure-methylester
4-(1-(1-Imidazolyl)-butyl)-benzoesäure-butylester
4-(1-(1-Imidazolyl)-butyl)-phenyl-acetonitril
4-(1-(1-Imidazolyl)-butyl)-benzaldehyd
4-(1-(1-Imidazolyl)-butyl)-benzylalkohol
1-(1-(4-Butoxymethylphenyl)-butyl)-imidazol
1-(1-(4-Phenoxymethylphenyl)-butyl)-imidazol
1-(1-(4-Methoxymethylphenyl)-butyl)-imidazol
4-(1-(1-Imidazolyl)-butyl)-benzoesäure, Hydrochlorid
4-(1-(1-Imidazolyl)-butyl)-benzoesäuredimethylamid
4′-(1-(1-Imidazolyl)-butyl)-propiophenon
4-(1-(1-Imidazolyl)-butyl)-benzanilid
4-(1-(1-Imidazolyl)-butyl)-benzoesäurepyrrolidid
1-(4-(1-(1-Imidazolyl)-butyl)-phenyl)-1-propanol
4-(1-(1-Imidazolyl)-butyl)-benzophenon
1-[4-(1-(1-Imidazolyl)-butyl)-benzyl]-pyrrolidin
1-(1-(4-Hydroxyiminomethylphenyl)-butyl)-imidazol
4′-(1-(1-Imidazolyl)-butyl)-trans-zimtsäure-äthylester
3-(1-(1-Imidazolyl)-butyl)-phenyl)-propionsäure-ethylester
1-(1-(4-Semicarbazonomethylphenyl)-butyl)-imidazol
1-(1-(4-Methoxyiminomethylphenyl)-butyl)-imidazol
4′-(1-(1-Imidazolyl)-butyl)-trans-zimtsäure, Hydrochlorid
3′-(1-(1-Imidazolyl)-butyl)-trans-zimtsäureethylester
3′-(1-(1-Imidazolyl)-butyl)--trans-zimtsäure
3-[3-(1-(1-Imidazolyl)-butyl)-phenyl]-propionsäure
4-[1-(1-Imidazolyl)-butyl]-benzoesäure
4-[1-(1-Imidazolyl)-butyl]-benzamid
1-<4-[1-(1-Imidazolyl)-butyl]-phenyl>-ethanol
4′-[1-(1-Imidazolyl)-butyl]-acetophenon
1-<4-[1-(1-Imidazolyl)-butyl]-phenyl>-2-methyl-1-propanol
<4-[1-(1-Imidazolyl)-butyl]-phenyl>-2-propyl-keton
<4-[1-(1-Imidazolyl)-butyl]-phenyl>-propylketon
<4-[1-(1-Imidazolyl)-butyl]-phenyl>-butylketon
<4-[1-(1-Imidazolyl)-butyl]-phenyl>-pentylketon
<4-[1-(1-Imidazolyl)-butyl]-phenyl>-hexylketon
Cyclohexyl-<4-[1-(1-Imidazolyl)-butyl]-phenyl>-keton
4′-[Cyclohexyl-(1-imidazolyl)-methyl]-acetophenon
N-Butyl-4-[1-(1-imidazolyl)-butyl]-benzamid
N-Cyclohexyl-4-[1-(1-imidazolyl)-butyl]-benzamid
N-p-Tolyl-4-[1-(1-imidazolyl)-butyl]-benzamid
N-(4-Chlorphenyl)-4-[1-(1-imidazolyl)-butyl]-benzamid
N-Benzyl-4-[1-(1-imidazolyl)-butyl]-benzamid
N-(3-Pyridyl)-4-[1-(1-imidazolyl)-butyl]-benzamid
4-[1-(1-Imidazolyl)-butyl]-benzoesäure-(4-methylpiperidid)

4-[1-(1-Imidazolyl)-butyl]-N-methyl-N-phenyl-benzamid
4-[1-(1-Imidazolyl)-butyl]-benzoesäure-(N′-methylpiperazid)
4-[1-(1-Imidazolyl)-butyl]-benzoesäure-isopropylester
4-[1-(1-Imidazolyl)-butyl]-benzoesäure-(1-methylbutyl)-ester
4-[1-(1-Imidazolyl)-butyl]-benzoesäure-cyclohexylester
4-[1-(1-Imidazolyl)-butyl]-benzoesäure-(2-methoxyethyl)-ester
4-[1-(1-Imidazolyl)-butyl]-benzoesäure-(2-methylmercaptoethyl)-ester
4-[1-(1-Imidazolyl)-butyl]-benzoesäure-(2-dimethylaminoethyl)-ester
4-[1-(1-Imidazolyl)-butyl]-benzoesäure-tert.-butylester
1-[1-(4-Ethoxyiminomethyl-phenyl)-butyl]-imidazol
1-[1-(4-Allyloxyiminomethyl-phenyl)-butyl]-imidazol
1-[1-(4-Benzyloxyiminomethyl-phenyl)-butyl]-imidazol
1-<1-[4-(4-Nitrobenzyloxyimino)-methyl-phenyl]-butyl>-imidazol
1-<1-[4-(2,3,4,5,6-Pentafluorbenzyloxyimino)-methyl-phenyl]-butyl>-imidazol
α-<4-[1-(1-Imidazolyl)-butyl]-phenyl>-benzylalkohol

3. Pharmazeutische Präparate, gekennzeichnet durch den Gehalt an mindestens einer Verbindung gemäß Anspruch 1 oder 2.

4. Verwendung der Verbindungen nach Anspruch 1 oder 2 zur Herstellung von Präparaten zur Behandlung von östrogen-bedingten Krankheiten.

5. Verfahren zur Herstellung von substituierten Imidazolen der allgemeinen Formel I gemäß Anspruch 1, dadurch gekennzeichnet, daß man

a) eine Verbindung der allgemeinen Formel II

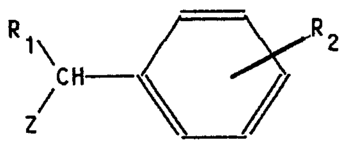

(II),

worin
$R_1$ und $R_2$ die in Anspruch 1 genannte Bedeutung haben und
Z eine Fluchtgruppe bedeutet,
mit einer Verbindung der allgemeinen Formel III

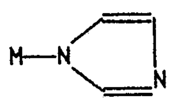

(III),

worin
M ein Wasserstoffatom oder ein Alkalimetallatom bedeutet,
umsetzt oder

b) eine Verbindung der allgemeinen Formel IV

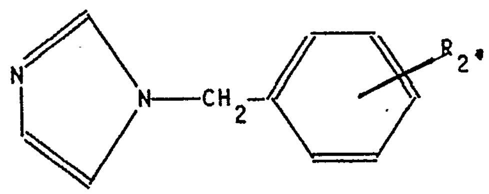

(IV),

worin
$R_{2'}$ dieselbe Bedeutung hat wie $R_2$ in Formel I mit der Maßgabe, daß $R_{2'}$ ein elektronenziehender, nicht deprotonierbarer Rest ist, in Gegenwart einer Base mit einer Verbindung der allgemeinen Formel V

$R_1$ - Z (V),

worin
$R_1$ die in Anspruch 1 genannte Bedeutung hat
und
Z eine Fluchtgruppe bedeutet,
umsetzt oder

c) eine Verbindung der allgemeinen Formel VI

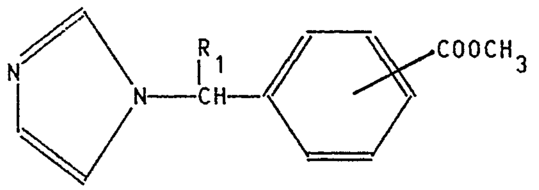

(VI),

worin

$R_1$ die in Anspruch 1 genannte Bedeutung hat,

mit einem Reduktionsmittel in die Hydroxymethylverbindung überführt, gegebenenfalls die Hydroxymethylverbindung mit einem Halogenierungsmittel zur Halogenmethylverbindung umsetzt, gegebenenfalls die Halogenmethylverbindung mit einem Metallcyanid in die Cyanomethylverbindung oder mit einem Metallalkoxid in die Alkoxymethylverbindung oder mit Ammoniak in die Aminomethylverbindung oder mit einem Amin zur substituierten Aminomethylverbindung, oder die Verbindung der allgemeinen Formel VI hydrolytisch in die Carboxylverbindung oder ins Carbonsäureamid oder unter Umesterungsbedingungen mit einem Alkohol in einen Carbonsäureester, gegebenenfalls die Carboxylverbindung mit einem Alkohol zum Carbonsäureester oder mit einem Halogenierungsmittel zum Carbonsäurehalogenid, gegebenenfalls das Carbonsäurehalogenid mit Ammoniak oder Aminen in Carbonsäureamide, gegebenenfalls die Hydroxymethylverbindung mit Braunstein in den Aldehyd, gegebenenfalls den Aldehyd mit Aminen oder substituierten Aminen in Imine oder substituierte Imine oder mit Wittigreagenzien in ungesättigte Verbindungen, gegebenenfalls die ungesättigten Verbindungen unter Hydrierungsbedingungen in gesättigte Verbindungen, gegebenenfalls den Aldehyd mit metallorganischen Verbindungen in die sekundären Alkohole und diese mit einem Oxidationsmittel in die substituierten Alkanoylderivate oder Benzoylderivate überführt.

Europäisches Patentamt

# EUROPÄISCHER RECHERCHENBERICHT

Nummer der Anmeldung

EP 89 73 0085

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 428 632 (KISSEI PHARMACEUTICAL CO., LTD ET ONO PHARMACEUTICAL CO., LTD)<br>* Insgesamt *<br>--- | 1-3,5 | C 07 D 233/60<br>A 61 K 31/415<br>C 07 D 233/58 |
| X | EP-A-0 003 732 (THE WELLCOME FOUNDATION LTD)<br>* Seiten 2-9 *<br>--- | 1,3,5 | |
| X | GB-A-2 054 567 (ASAHI KASEI KOGYO)<br>* Insgesamt *<br>----- | 1,5 | |
| | | | RECHERCHIERTE SACHGEBIETE (Int. Cl.4) |
| | | | C 07 D 233/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 05-07-1989 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument